# EUROPEAN PATENT SPECIFICATION

(11) **EP 1 001 761 B1**
(45) Date of publication and mention of the grant of the patent: **28.07.2004**
(21) Application number: 98938759.2
(22) Date of filing: 07.08.1998
(51) Int. Cl.: A61K 31/27, A61K 31/66, A61K 31/44, A61K 31/4406, A61K 31/40, A61K 31/435, A61K 31/55, A61P 25/00

(54) **USE OF CHOLINESTERASE INHIBITORS FOR TREATING ATTENTION DEFICIT DISORDERS**
VERWENDUNG VON CHOLINESTERASEHEMMERN ZUR BEHANDLUNG VON AUFMERKSAMKEITSMANGEL-STÖRUNGEN
UTILISATION D'INHIBITEURS DE CHOLINESTERASE DANS LE TRAITEMENT DES TROUBLES DEFICITAIRES DE L'ATTENTION

(30) Priority: 08.08.1997 GB 9716879
(43) Date of publication of application: 24.05.2000
(73) Proprietor: Snorrason, Ernir, 105 Reykjavik (IS)
(72) Inventor: SNORRASON, Ernir, IS-105 Reykjavik (IS); MURRAY, James, Robert, Shaftesbury, Dorset SP7 9ND (GB)
(74) Representative: Tubby, David George
(86) International application number: PCT/GB1998/002378
(87) International publication number: WO 1999/007359

(56) References cited:
- EP-A- 0 193 926
- EP-A- 0 229 391
- EP-A- 0 411 534
- EP-A- 0 515 302
- EP-A- 0 607 864
- WO-A-92/20328
- WO-A-95/29909
- WO-A-97/46527
- WO-A-98/39000
- US-A- 4 550 113
- E.D. LEVIN ET AL.: "NICOTINE EFFECTS ON ADULTS WITH ATTENTION DEFICIT/HYPERACTIVITY DISORDER" PSYCHOPHARMACOLOGY, vol. 123, no. 1, 1996, pages 55-63, XP002087140
- J.L. JUNCOS ET AL.: "CHOLINERGIC STRATEGIES IN TOURETTE SYNDROME: AN OPEN-LABEL TRIAL OF TACRINE HYDROCHLORIDE" NEUROLOGY, vol. 48, no. 3S.2, March 1997, page A397 XP002087141
- B.J. SAHAKIAN ET AL.: "NICOTINE AND TETRAHYDROAMINOACRIDINE: EVIDENCE FOR IMPROVED ATTENTION IN PATIENTS WITH DEMENTIA OF THE ALZHEIMER TYPE" DRUG DEVELOPMENT RESEARCH, vol. 31, no. 1, 1994, pages 80-88, XP002087142
- KIRKBY D L ET AL: "EFFECTS OF ANTICHOLINESTERASE DRUGS TACRINE AND 32020, THE 5-HT3 ANTAGONIST ONDANSETRON, AND THE H3 ANTAGONIST THIOPERAMIDE, IN MODELS OF COGNITION AND CHOLINERGIC FUNCTION" BEHAVIOURAL PHARMACOLOGY, vol. 7, no. 6, November 1996, pages 513-525, XP002068916
- J.L. MUIR ET AL.: "REVERSAL OF VISUAL ATTENTIONAL DYSFUNCTION FOLLOWING LESIONS OF THE CHOLINERGIC BASAL FOREBRAIN BY PHYSOSTIGMINE AND NICOTINE BUT NOT BY THE 5-HT3 RECEPTOR ANTAGONIST, ONDANSETRON" PSYCHOPHARMACOLOGY, vol. 118, no. 1, 1995, pages 82-92, XP002087143
- D.N.C. JONES ET AL.: "AGE-ASSOCIATED IMPAIRMENTS IN A TEST OF ATTENTION: EVIDENCE FOR INVOLVEMENT OF CHOLINERGIC SYSTEMS" JOURNAL OF NEUROSCIENCE, vol. 15, no. 11, 1995, pages 7282-7292, XP002087144
- C. KERTZMAN ET AL.: "EFFECTS OF PHYSOSTIGMINE ON SPATIAL ATTENTION IN PATIENTS WITH PROGRESSIVE SUPRANUCLEAR PALSY" ARCHIVES OF NEUROLOGY, vol. 47, no. 12, 1990, pages 1346-1350, XP002087145
- RILEY E P ET AL: "THE EFFECTS OF PHYSOSTIGMINE ON OPEN-FIELD BEHAVIOR IN RATS EXPOSEDTO ALCOHOL PRENATALLY" ALCOHOLISM: CLINICAL AND EXPERIMENTAL RESEARCH, vol. 10, no. 1, January 1986, pages 50-53, XP000612593

## Description

The present invention relates to combatting attention deficit disorders (hereinafter referred to as ADD). In particular, the invention relates to the use of certain pharmaceutically acceptable cholinesterase inhibitors in the manufacture of a medicament for use in combatting ADD.

Attention deficit disorders affect children, adolescents and adults affecting up to 10% of children and at least 2% of adults. The childhood condition is often known as attention-deficit hyperactivity disorder (ADHD). Symptoms usually first manifest between the ages of 2 - 5. These children have a short attention span, motor hyperactivity, and are disorganised, forgetful and impulsive. ADHD can have a serious effect on the child's academic progress and performance and causes stress to family and teachers and difficulties in interacting with the child's peer group. The condition can persist into adult life. In adults, the major symptoms include distractibility, disorganisation and accident proneness. The condition in adults and children causes serious disruption to normal lifestyle patterns, and creates great stresses to all aspects of daily life, in the home, the workplace and in social situations. ADD as used herein covers both the adult and child forms of the condition, as well as hyperkinetic disorders and disorders which include any of the characterising criteria of the American Psychiatric Association's DSM-IV Classification of the disorders.

Various therapies have been proposed but have not proved satisfactory, due inter alia to lack of efficacy and/or undesirable side effects. Stimulants such as dexamphetamine, methylphenidate and pemoline have been used clinically in the management of ADD. Whilst they have been found to improve the primary manifestations such as motor activity, distractability and impulsivity in some sufferers, their side effects including anorexia, delayed onset of sleep, mood changes and exacerbation of pre-existing psychotic symptoms together with the possible potential for abuse, limits their utility. Other classes of drug such as tricyclic antidepressants have been suggested in the literature but are not generally regarded as being effective agents for the management of ADD.

WO-A-92 20328 discloses the use of cholinesterase inhibitors including galantamine for counteracting the sedative, hypnotic or respiratory depressive side effects of benzodiazepines when benzodiazepines are used to treat certain diseases, for example hyperactivity in children.

J. L. Juncos et al., Neurology, 1997, 48, page A397 notes improvements in certain attention deficit disorder symptoms in a trial of tacrine on Tourette Syndrome patients, and suggests the use of tacrine as an alternative to dopamine blockers for treating Tourette Syndrome.

WO-A-95 29909 discloses certain amide compounds for enhancing the release of neurotransmitters such as acetylcholine, dopamine and serotonin, and suggests their use in treating diseases treatable by enhancing acetylcholine, dopamine or serotonin release, for example attention deficit disorder.

EP-A-0 229 391 discloses certain piperidine derivatives having anticholinesterase activity, and suggests their use as medicines based on this activity, for example in treating attention disturbances.

EP-A-0 411 534 discloses certain tacrine derivatives and suggests their use in treating conditions of cerebral insufficiency characterised by decreased cerebral acetylcholine production or release, for example hyperkinesia.

EP-A-0 607 864 discloses tricyclic compounds having acetylcholinesterase inhibitory activity, and suggests their use in treating cholinesterase dependent diseases, for example hyperkinesia.

There is accordingly a need for a new therapeutic treatment for the management of ADD. The present invention provides such a treatment.

Thus we have surprisingly found that cholinesterase inhibitors such as galantamine can effectively combat ADD.

According to one aspect, the present invention provides a method of combatting ADD comprising administering to a subject a pharmaceutically acceptable cholinesterase inhibitor.

Accordingly, the invention provides the use of a pharmaceutically acceptable galantamine compound of formula I or II in the manufacture of a medicament for combatting ADD.

As used herein the term 'combatting' includes both therapy and prophylaxis.

The invention encompasses the use of any cholinesterase inhibitor of formula I or II, provided of course that it is pharmaceutically acceptable.

Examples of cholinesterase inhibitors which may be used according to the invention include pro-drugs of any of these in which the inhibitor is modified in accordance with principles of pro-drug construction known in the art. Examples of such modifications include the introduction of hydrophilic or lipophilic groups to enhance solubility, or penetration through cell membranes, respectively.

Preferred for use according to the invention are acetylcholinesterase inhibitors of formula I or II, particularly those which are capable of crossing the blood brain barrier.

Particularly preferred cholinesterase inhibitors of formula I or II for use according to the invention include galantamine, epigalantamine and norgalantamine, and analogues, salts and derivatives of any of these. Galantamine was previously known as galanthamine. It is a tertiary alkaloid which can be extracted from various snowdrop bulbs e.g. the Caucasian snowdrop galanthus woronowii(Amaryllidaceae) and related species and daffodil bulbs or made by chemical synthesis. It has a high selectivity for acetylcholinesterase as opposed to butyrylcholinesterase. It is active substantially selectively at nicotinic receptor sites with substantially little effect on muscarinic receptor sites.

The cholinesterase inhibitors for use in the invention are galantamine and its derivatives of formula (I): 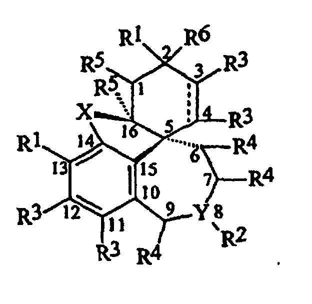 wherein the broken line represents an optionally present double bond between carbon atoms 3 and 4, each R₁ is independently selected from hydrogen, hydroxyl, straight or branched chain alkyl, hydroxyalkyl, carboxyalkyl amino, alkylamino, acyl, lower alkanoyl, cyano, sulfhydryl, C₁₋₆alkoxy, alkylthio, aryloxy, arylthio, R₃-substituted aryloxy, R₃-substituted arylthio, aralkoxy, an optionally R₃-substituted aliphatic or aryl carbamyl group, aralkylthio, R₃-substituted aralkoxy, R₃-substituted aralkylthio, aryloxymethyl, R₃-substituted aryloxymethyl, alkanoyloxy, hydroxy-substituted alkanoyloxy, benzoyloxy, R₃-substituted benzoyloxy, aryloxycarbonyl and R₃-substituted aryloxycarbonyl,

R₂ is selected from hydrogen, straight or branched chain C₁₋₆alkyl, alkenyl or alkaryl group, optionally substituted by a halogen atom or a cycloalkyl, hydroxy, alkoxy, nitro, amino, aminoalkyl, acylamino, heteroaryl, heteroaryl-alkyl, aryl, arylalkyl, cyano, amyl, aroyl, cycloalkylmethyl, allyl, phenyl, R₃-substituted phenyl, alkylphenyl, R₃-substituted alkylphenyl, heterocyclyl selected from α- or β-furyl, α- or β-thienyl, thenyl, pyridyl, pyrazinyl, and pyrimidyl, alkyl-heterocyclyl or R'-substituted heterocyclyl, where R' is alkyl or alkoxy,
each R₃ is independently selected from hydrogen, hydroxyl, sulfhydryl, alkyl, hydroxyalkyl, aryl, aralkyl, alkoxy, mercaptoalkyl, aryloxy, thiaryloxy, alkaryloxy, mercaptoalkaryl, nitro, amino, N-alkylamino, N-arylamino, N-alkarylamino, fluoro, chloro, bromo, iodo, and trifluoromethyl,
each R₄ is independently selected from hydrogen, halo, trifluoromethyl or C₁₋₄-alkyl,
each R₅ is independently selected from hydrogen or hydroxymethyl,
R₆ is hydrogen or C₁₋₆alkyl, or when R₁ at carbon atom 2 is hydroxyl, R₆ may be a moiety of formula I wherein R₆ is hydrogen and R₁ is a linking bond; or
R₁ at carbon atom 2 and R₆ may jointly form semicarbazone,
X is oxygen or NR₃,
Y is nitrogen or phosphorus,
and methylenedioxy derivatives thereof and
pharmaceutically acceptable acid addition salts thereof.

Of the compounds of formula I which may be used in the method of the invention, preferred compounds are those in which the alkyl moieties contain 1 to 8 carbon atoms, halogen atoms are preferably fluorine, bromine, chlorine, aryl moieties are preferably phenyl, cycloalkyl groups are preferably 3- to 7-membered rings, especially cyclopropyl or cyclobutyl, acyl groups are preferably lower alkanoyl groups and heteroaryl moieties are preferably 5- to 8-membered rings, e.g., thienyl, furyl, pyridyl, pyrrolyl, or pyrizanyl.

Preferred compounds of formula I are the compounds of formula II 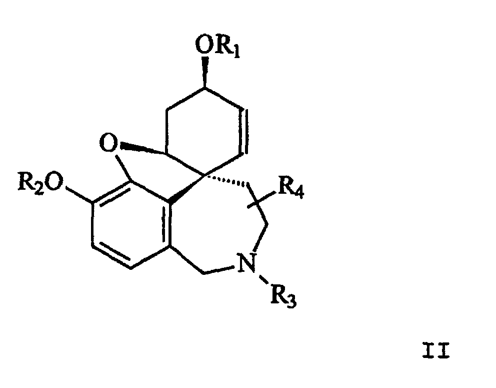 wherein R¹ and R² which may be the same or different each represents a hydrogen atom or an acyl group, such as a lower alkanoyl group, e.g. an acetyl group or a straight-chained or branched alkyl group, e.g. methyl, ethyl, propyl, or isopropyl;
R³ is a straight or branched chain alkyl, alkenyl or alkaryl group which is optionally substituted by a halogen atom or a cycloalkyl, hydroxy, alkoxy, nitro, amino, aminoalkyl, acylamino, heteroaryl, heteroarylalkyl, aroyl, aroylalkyl or cyano group; and
R⁴ represents a hydrogen or a halogen atom attached to at least one of the ring carbons of the tetracyclic skeleton,
and pharmaceutically acceptable salts thereof, such as a hydrobromide, hydrochloride, methylsulphate or methiodide.

Formula II includes galantamine itself.

Particularly preferred is galantamine itself, and salts thereof such as halides for example galantamine hydrobromide and the use of these compounds in the manufacture of a medicament for combatting ADD provides a further aspect of the invention.

Among these compounds are those described in EP-A-236684 and WO88/08708, the disclosures of which are incorporated herein by reference. Galantamine and its derivatives of formula I and II may be prepared by the methods described in these publications.

The cholinesterase inhibitors of formula I or II for use in the invention include compounds which are functionally similar to galantamine. These are defined herein as compounds which possess an at least 10-fold selectivity, preferably an at least 20-fold selectivity, more preferably an at least 40-fold selectivity, and most preferably an at least 50 fold selectivity, for acetylcholinesterase as opposed to butyrylcholinesterase, when measured by the in vitro method of Thomsen and Kewitz: Selective Inhibition of Human Acetylcholinesterase by Galantamine in vitro and in vivo, Life Sciences, Vol 46, pp. 1553-1558 (1990), and T. Thomsen, H. Kewitz and O. Pleul, J. Clin. Chem. Clin. Biochem. 26 469-475 (1988). The in vitro test described by Thomsen and Kewitz in Life Sciences, Vol 46, pp 1553-1558 (1990) is the one referred to herein whenever numeric (10-fold, 20-fold, 40-fold) reference to selectivity for acetylcholinesterase as opposed to butyrylcholinesterase is made. According to Thomsen and Kewitz, galantamine hydrobromide, when tested under the conditions described, shows a 50-fold selectivity; this selectivity value is taken as the "fix-point" whenever in vitro selectivities are discussed herein and could be used, for the purpose of determining the selectivities for other cholinesterase inhibitors of formula I or II, as a calibration value which is the one to establish with galantamine hydrobromide in any repetition of the experiment described by Thomsen and Kewitz. Thus, with reference to this determination method, a preferred acetylcholinesterase inhibitor is one which in the in vitro method described has an at least 10-fold selectivity for acetylcholinesterase as opposed to butyrylcholinesterase, such as an at least 20-fold selectivity for acetylcholinesterase as opposed to butyrylcholinesterase, e.g. an at least 40-fold selectivity for acetylcholinesterase as opposed to butyrylcholinesterase. A selectivity test is commercially available (from Sigma Diagnostics).

For use in the invention the cholinesterase inhibitor such as galantamine and derivatives and salts thereof may be formulated according to conventional methods of pharmacy, together where appropriate with one or more pharmaceutically acceptable carriers, excipients or diluents such as, for example, are described in Remingtons Pharmaceutical Sciences. Such formulations may for example take the form of tablets, capsules, solutions, or lozenges, pessaries, creams, suppositories or transdermal formulations such as patches, creams, ointments or lotions, depending upon the administration route to be used, which may include enterally or parenterally, including orally or injection via the intravenous, intramuscular or subcutaneous routes, or intrathecally by means of an implanted device.

Oral and transdermal administration routes are preferred.

Precise dosage rates and regimes will depend upon the individual patient and may be determined by the medical practitioner based on individual circumstances. For oral administration doses may be within the range of 5-100 mg per day, such as 2 to 70 mg per day eg. 10 to 30 mg. For transdermal administration galanthamine may be delivered in equivalent daily doses. For parenteral administration, dosages may be in the range of 0.1 to 100 mg per day, such as 5 to 100 mg per day, e.g. 10 to 50 mg per day, including 5 to 30 mg per day; lower dosages are often preferred.

Galantamine and its acid addition salts form crystals. They are generally only sparingly soluble in water at room temperature; therefore, injectable compositions are normally in the form of an aqueous suspension. If necessary, pharmaceutically-acceptable suspension aids may be employed. Typically, such a suspension will be employed at a concentration of 0.1-50 mg/ml, such as 1-50 mg/ml, more commonly 5-40 mg/ml, for example, 5-30 mg/ml or 10-40 mg/ml, such as 10-30 mg/ml, especially 20-30 mg/ml of galantamine.

Cholinesterase inhibitors such as galantamine and salts thereof may be used as the sole drug in the management of ADD, or may be used together with other agents useful in managing ADD.

The invention will now be described with reference to the following non-limiting examples.

### Example 1

### Formulation of tablets containing galantamine

| Composition of 1 tablet containing 1 mg galantamine | |
|---|---|
| Galantamine hydrobromide | 0.001 g |
| Calcium phosphate | 0.032 g |
| Lactose | 0.005 g |
| Wheat Starch | 0.0056 g |
| Microcrystalline Cellulose | 0.015 g |
| Talc | 0.0007 g |
| Magnesium Stearate | 0.0007 g |

| Composition of 1 tablet containing 5 mg galantamine | |
|---|---|
| Galantamine hydrobromide | 0.005 g |
| Calcium phosphate | 0.024 g |
| Lactose | 0.004 g |
| Wheat Starch | 0.004 g |
| Microcrystalline Cellulose | 0.04 g |
| Talc | 0.002 g |
| Magnesium Stearate | 0.001 g |

| Composition of 1 tablet containing 10 mg galantamine | |
|---|---|
| Galantamine hydrobromide | 0.010 g |
| Lactose | 0.040 g |
| Wheat Starch | 0.0234 g |
| Microcrystalline Cellulose | 0.0374 g |
| Talc | 0.0036 g |
| Magnesium Stearate | 0.0012 g |
| Gelatin | 0.0044 g |

### Preparation

All the tablets are prepared according to routine tabletting procedures.

### Example 2

### Treatment of patients suffering from ADD

Three patients aged 9 to 11 years diagnosed as having ADD were treated with 5 mg of galantamine given 3 times per day for a total of 14 days. After this period of time, the patients showed marked improvement of symptoms, particularly as regards attention and restlessness, as judged on an ecological behavioural scale (translated: Barkley, RA et al, Assessing situational variation in children's problem behaviours: The Home and School Situations Questionnaires, in RJ Prinz (Ed), Advances in Behavioural Assessment of Children and Families, vol 3, pp 157-176, Greenwich, CT, JAI Press, Inc).

A further cohort of two 5 year old patients with ADD have been treated and again symptoms have generally improved, especially attention, as measured by ANT (Amsterdam Neuropsychological Tests), which are standard tests that measure reaction times for different tasks.

The protocol in this case is first a baseline testing before medication and retesting after 2 days on medication and then after a week repeated testing. Ecological scales were also used before and are now being evaluated after one week after two weeks etc. for a treatment of one month duration. The following table shows a selection of results obtained for these two children before and after treatment with galantamine.

| | Before: | | | | After: | | | |
|---|---|---|---|---|---|---|---|---|
| | mean | Sd | miss¹ | Fa² | mean | Sd | miss | Fa |
| **Patient A** | | | | | | | | |
| Baseline: | 685 | 766 | | | 727 | 470 | | |
| GoNoGo | 705 | 158 | 1 | 1 | 655 | 173 | 1 | 2 |
| Sustained Att. | 1302 | 565 | 14 | 13 | 1222 | 472 | 4 | 6 |
| Focused Att. | 1214 | 404 | | | 1352 | 434 | 0 | |
| | | | | | | | | |

| **Patient B** | | | | | | | | |
|---|---|---|---|---|---|---|---|---|
| Baseline: | 1051 | 899 | | | 780 | 418 | | |
| GoNoGo | 804 | 162 | 2 | 9 | 766 | 172 | 3 | 3 |
| Sustained Att. | 1444 | 721 | 31 | 29 | 1152 | 600 | 20 | 50 |
| Focused Att. | 2444 | 1367 | 1 | | 1166 | 270 | 3 | |

| | | | | | | | | |
|---|---|---|---|---|---|---|---|---|
| ¹ Missed task | | | | | | | | |
| ² Failed attempt | | | | | | | | |

This data was normalized and submitted to error analysis that showed an improvement in attention (reduction in time (msec) to complete certain tasks and with fewer errors) over 2.5 standard deviations after medication with galantamine hydrobromide 2.5 mg 3 times a day.

## Claims

1. The use of galantamine or a derivative thereof of formula I: 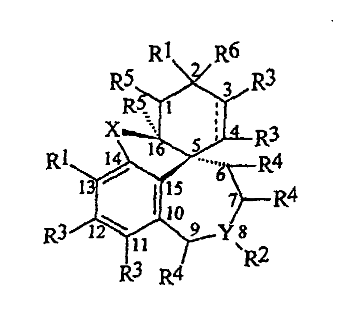 wherein the broken line represents an optionally present double bond between carbon atoms 3 and 4, each R₁ is independently selected from hydrogen, hydroxyl, straight or branched chain alkyl, hydroxyalkyl, carboxyalkyl amino, alkylamino, acyl, lower alkanoyl, cyano, sulfhydryl, C₁₋₆alkoxy, alkylthio, aryloxy, arylthio, R₃-substituted aryloxy, R₃-substituted arylthio, aralkoxy, an optionally R₃-substituted aliphatic or aryl carbamyl group, aralkylthio, R₃-substituted aralkoxy, R₃-substituted aralkylthio, aryloxymethyl, R₃-substituted aryloxymethyl, alkanoyloxy, hydroxy-substituted alkanoyloxy, benzoyloxy, R₃-substituted benzoyloxy, aryloxycarbonyl and R₃-substituted aryloxycarbonyl,
R₂ is selected from hydrogen, straight or branched chain C₁₋₆alkyl, alkenyl or alkaryl group, optionally substituted by a halogen atom or a cycloalkyl, hydroxy, alkoxy, nitro, amino, aminoalkyl, acylamino, heteroaryl, heteroaryl-alkyl, aryl, arylalkyl, cyano, amyl, aroyl, cycloalkylmethyl, allyl, phenyl, R₃-substituted phenyl, alkylphenyl, R₃-substituted alkylphenyl, heterocyclyl selected from α- or β-furyl, α- or β-thienyl, thenyl, pyridyl, pyrazinyl, and pyrimidyl, alkyl-heterocyclyl or R'-substituted heterocyclyl, where R' is alkyl or alkoxy,
each R₃ is independently selected from hydrogen, hydroxyl, sulfhydryl, alkyl, hydroxyalkyl, aryl, aralkyl, alkoxy, mercaptoalkyl, aryloxy, thiaryloxy, alkaryloxy, mercaptoalkaryl, nitro, amino, N-alkylamino, N-arylamino, N-alkarylamino, fluoro, chloro, bromo, iodo, and trifluoromethyl,
each R₄ is independently selected from hydrogen, halo, trifluoromethyl or C₁₋₄-alkyl,
each R₅ is independently selected from hydrogen or hydroxymethyl,
R₆ is hydrogen or C₁₋₆alkyl, or when R₁ at carbon atom 2 is hydroxyl, R₆ may be a moiety of formula I wherein R₆ is hydrogen and R₁ is a linking bond; or
R₁ at carbon atom 2 and R₆ may jointly form semicarbazone,
X is oxygen or NR₃,
Y is nitrogen or phosphorus,
and methylenedioxy derivatives thereof and
pharmaceutically acceptable acid addition salts thereof
in the manufacture of a medicament for combatting attention deficit disorders.

2. The use of galantamine or a derivative thereof of formula II 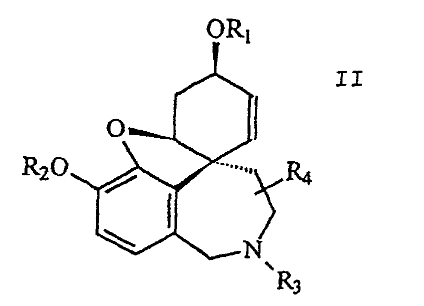 wherein R¹ and R² which may be the same or different each represents a hydrogen atom or an acyl group, such as a lower alkanoyl group, e.g. an acetyl group or a straight-chained or branched alkyl group, e.g. methyl, ethyl, propyl, or isopropyl;
R³ is a straight or branched chain alkyl, alkenyl or alkaryl group which is optionally substituted by a halogen atom or a cycloalkyl, hydroxy, alkoxy, nitro, amino, aminoalkyl, acylamino, heteroaryl, heteroarylalkyl, aroyl, aroylalkyl or cyano group; and
R⁴ represents a hydrogen or a halogen atom attached to at least one of the ring carbons of the tetracyclic skeleton,
and pharmaceutically acceptable salts thereof, such as a hydrobromide, hydrochloride, methylsulphate or methiodide in the manufacture of a medicament for combatting attention deficit disorders.

3. The use of galantamine or a salt thereof in the manufacture of a medicament for combatting attention deficit disorders.

4. The use as claimed in any one of claims 1 to 3 wherein the disorder is attention deficit hyperactivity disorder.

5. The use as claimed in any one of claims 1 to 3 wherein the disorder is a hyperkinetic disorder.

## Patentansprüche

1. Verwendung von Galanthamin oder eines Derivats davon der Formel I: 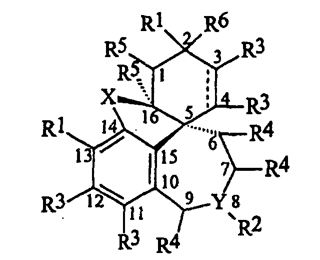 worin die gestrichelte Linie eine wahlweise vorhandene Doppelbindung zwischen den Kohlenstoffatomen 3 und 4 darstellt, jedes R₁ ist unabhängig ausgewählt aus Wasserstoff, Hydroxyl, geradkettiges oder verzweigtes Alkyl, Hydroxyalkyl, Carboxyalkylamino, Alkylamino, Acyl, niederes Alkanoyl, Cyano, Sulfhydryl, C₁₋₆-Alkoxy, Alkylthio, Aryloxy, Arylthio, R₃-substituiertes Aryloxy, R₃-substituiertes Arylthio, Aralkoxy, eine wahlweise R₃-substituiertes aliphatisches oder Arylcarbamyl-Gruppe, Aralkylthio, R₃-substituiertes Aralkoxy, R₃-substituiertes Aralkylthio, Aryloxymethyl, R₃-substituiertes Aryloxymethyl, Alkanoyloxy, Hydroxy-substituiertes Alkanoyloxy, Benzoyloxy, R₃-substituiertes Benzoyloxy, Aryloxycarbonyl und R₃-substituiertes Aryloxycarbonyl;
R₂ ist ausgewählt aus Wasserstoff, einer geradkettigen oder verzweigten C₁₋₆-Alkyl-, Alkenyl- oder Alkaryl-Gruppe, wahlweise substituiert durch ein Halogenatom oder ein Cycloalkyl, Hydroxy, Alkoxy, Nitro, Amino, Aminoalkyl, Acylamino, Heteroaryl, Heteroarylalkyl, Aryl, Arylalkyl, Cyano, Amyl, Aroyl, Cycloalkylmethyl, Allyl, Phenyl, R₃-substituiertes Phenyl, Alkylphenyl, R₃-substituiertes Alkylphenyl, Heterocyclyl, ausgewählt aus α- oder β-Furyl, α- oder β-Thienyl, Thenyl, Pyridyl, Pyrazinyl und Pyrimidyl, Alkylheterocyclyl oder R'-substituiertes Heterocyclyl, worin R' Alkyl oder Alkoxy ist;
jedes R₃ ist unabhängig ausgewählt aus Wasserstoff, Hydroxyl, Sulfhydryl, Alkyl, Hydroxylalkyl, Aryl, Aralkyl, Alkoxy, Mercaptoalkyl, Aryloxy, Thiaryloxy, Alkaryloxy, Mercaptoalkaryl, Nitro, Amino, N-Alkylamino, N-Arylamino, N-Alkarylamino, Fluor, Chlor, Brom, Iod und Trifluormethyl;
jedes R₄ ist unabhängig ausgewählt aus Wasserstoff, Halogen, Trifluormethyl oder C₁₋₄-Alkyl;
jedes R₅ ist unabhängig ausgewählt aus Wasserstoff oder Hydroxymethyl;
R₆ ist Wasserstoff oder C₁₋₆-Alkyl oder, wenn R₁ am Kohlenstoffatom 2 Hydroxyl ist, kann R₆ ein Teil von Formel I sein, worin R₆ Wasserstoff ist und R₁ eine verknüpfende Bindung ist; oder
R₁ am Kohlenstoffatom 2 und R₆ kann gemeinsam ein Semicarbazon bilden;
X ist Sauerstoff oder NR₃;
Y ist Stickstoff oder Phosphor;
und Methylendioxy-Derivate davon sowie pharmazeutisch zulässige SäureAdditionssalze davon,
in der Herstellung eines Medikaments für die Bekämpfung der Aufmerksamkeitsdefizitsyndrome.

2. Verwendung von Galanthamin oder einem Derivat davon der Formel II 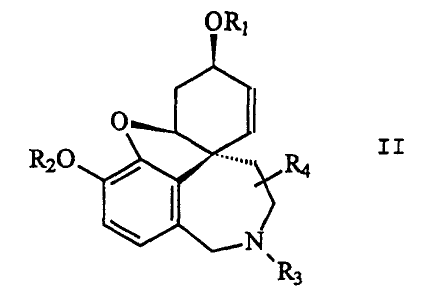 worin R¹ und R², gleich oder verschieden sein können, jedes ein Wasserstoffatom oder eine Acyl-Gruppe darstellen, wie beispielsweise eine niedere Alkanoyl-Gruppe, z.B. eine Acetyl-Gruppe oder eine geradkettige oder verzweigte Alkyl-Gruppe, z.B. Methyl, Ethyl, Propyl oder Isopropyl;
R³ ist eine geradkettige oder verzweigte Alkyl-, Alkenyl- oder Alkaryl-Gruppe, die wahlweise durch ein Halogenatom substituiert ist oder durch eine Cycloalkyl-, Hydroxy-, Alkoxy-, Nitro-, Amino-, Aminoalkyl-, Acylamino-, Heteroaryl-, Heteroarylalkyl-, Aroyl-, Aroylalkyl- oder Cyano-Gruppe; und
R⁴ stellt ein Wasserstoff oder ein Halogenatom dar, das an mindestens einem der Ringkohlenstoffatome des tetracyclischen Gerüsts angebracht ist;
sowie pharmazeutisch zulässige Salze davon, wie beispielsweise ein Hydrobromid, Hydrochlorid, Methylsulfat oder Methiodid in der Herstellung eines Medikaments für die Bekämpfung der Aufmerksamkeitsdefizitsyndrome.

3. Verwendung von Galanthamin oder eines Salzes davon in der Herstellung eines Medikaments für die Bekämpfung der Aufmerksamkeitsdefizitsyndrome.

4. Verwendung nach einem der Ansprüche 1 bis 3, wobei die Erkrankung das Aufmerksamkeitsdefizitsyndrom mit Hyperaktivität ist.

5. Verwendung nach einem der Ansprüche 1 bis 3, wobei die Erkrankung ein hyperkinetisches Syndrom ist.

## Revendications

1. Utilisation de galantamine ou d'un dérivé de celle-ci de la formule I: 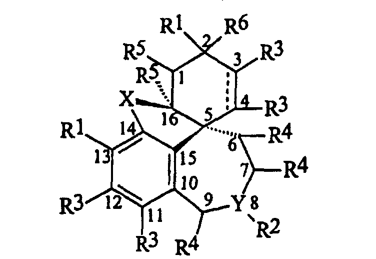 dans laquelle la ligne en pointillés représente une liaison double éventuellement présente entre les atomes de carbone 3 et 4, chaque R₁ est indépendamment choisi parmi un hydrogène, un groupe hydroxyle, alkyle à chaîne droite ou ramifiée, hydroxyalkyle, carboxyalkyle, amino, alkylamino, acyle, alcanoyle inférieur, cyano, sulfhydryle, alkoxy C₁₋₆, alkylthio, aryloxy, arylthio, aryloxy substitué par R₃, arylthio substitué par R₃, aralkoxy, un groupe carbamyle aliphatique ou aryle éventuellement substitué par R₃, aralkylthio, aralkoxy substitué par R₃, aralkylthio substitué par R₃, aryloxyméthyle, aryloxyméthyle substitué par R₃, alcanoyloxy, alcanoyloxy substitué par un groupe hydroxyle, benzoyloxy, benzoyloxy substitué par R₃, aryloxycarbonyle et aryloxycarbonyle substitué par R₃,
R₂ est choisi parmi un hydrogène, un groupe alkyle C₁₋₆ à chaîne droite ou ramifiée, un groupe alcényle ou alkaryle, éventuellement substitué par un atome d'halogène ou un groupe cycloalkyle, hydroxyle, alkoxy, nitro, amino, aminoalkyle, acylamino, hétéroaryle, hétéroarylalkyle, aryle, arylalkyle, cyano, amyle, aroyle, cycloalkylméthyle, allyle, phényle, phényle substitué par R₃, alkylphényle, alkylphényle substitué par R₃, hétérocyclyle choisi parmi un groupe α- ou β-furyle, α- ou β-thiényle, thényle, pyridyle, pyrazinyle et pyrimidyle, alkylhétérocyclyle ou hétérocyclyle substitué par R', où R' est un groupe alkyle ou alkoxy,
chaque R₃ est indépendamment choisi parmi un hydrogène, un groupe hydroxyle, sulfhydryle, alkyle, hydroxyalkyle, aryle, aralkyle, alkoxy, mercaptoalkyle, aryloxy, thiaryloxy, alkaryloxy, mercaptoalkaryle, nitro, amino, N-alkylamino, N-arylamino, N-alkarylamino, fluoro, chloro, bromo, iodo et trifluorométhyle,
chaque R₄ est indépendamment choisi parmi un hydrogène, un groupe halo, trifluorométhyle ou alkyle C₁₋₄,
chaque R₅ est indépendamment choisi parmi un hydrogène ou un groupe hydroxyméthyle,
R₆ est un hydrogène ou un groupe alkyle C₁₋₆ ou, lorsque R¹ à l'atome de carbone 2 est un groupe hydroxyle, R₆ peut être une fraction de la formule I dans laquelle R₆ est un hydrogène et R₁ est une liaison, ou
R¹ à l'atome de carbone 2 et R⁶ peut former ensemble une semicarbazone,
X est un oxygène ou NR³,
Y est un azote ou un phosphore,
et dérivés méthylènedioxy de celle-ci et sels d'addition acides pharmaceutiquement acceptables de celle-ci,
dans la fabrication d'un médicament pour combattre des troubles de déficience de l'attention.

2. Utilisation de galantamine ou d'un dérivé de celle-ci de la formule II: 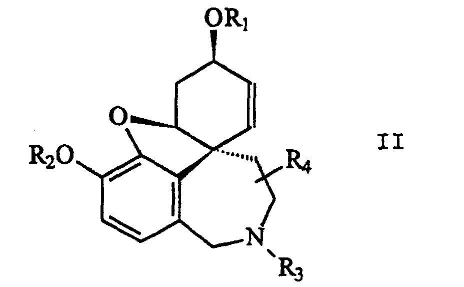 dans laquelle R¹ et R² qui peuvent être identiques ou différents représentent chacun un atome d'hydrogène ou un groupe acyle, tel qu'un groupe alcanoyle inférieur, par ex. un groupe acétyle ou un groupe alkyle à chaîne droite ou ramifiée, par ex. un groupe méthyle, éthyle, propyle ou isopropyle;
R³ est un groupe alkyle à chaîne droite ou ramifiée, alcényle ou alkaryle qui est éventuellement substitué par un atome d'halogène ou un groupe cycloalkyle, hydroxyle, alkoxy, nitro, amino, aminoalkyle, acylamino, hétéroaryle, hétéroarylalkyle, aroyle, aroylalkyle ou cyano; et
R⁴ représente un atome d'hydrogène ou d'halogène attaché à au moins un des carbones de cycle du squelette tétracyclique,
et sels pharmaceutiquement acceptables de celle-ci, tels qu'un bromhydrate, un chlorhydrate, un méthylsulfate ou un méthiodure dans la fabrication d'un médicament pour combattre des troubles de déficience de l'attention.

3. Utilisation de galantamine ou d'un sel de celle-ci dans la fabrication d'un médicament pour combattre des troubles de déficience de l'attention.

4. Utilisation suivant l'une quelconque des revendications 1 à 3, dans laquelle le trouble est un trouble de déficience de l'attention d'hyperactivité.

5. Utilisation suivant l'une quelconque des revendications 1 à 3, dans laquelle le trouble est un trouble hyperkinétique.
